# EUROPEAN PATENT SPECIFICATION

(11) **EP 1 217 958 B1**
(45) Date of publication and mention of the grant of the patent: **04.07.2007**
(21) Application number: 00968866.4
(22) Date of filing: 06.10.2000
(51) Int. Cl.: A61B 17/28

(54) **SURGICAL GRASPING DEVICE AND COMPONENTS THEREOF**
CHIRURGISCHES GREIFGERÄT UND KOMPONENTEN DAVON
DISPOSITIF CHIRURGICAL DE PREHENSION ET COMPOSANTS CORRESPONDANTS

(30) Priority: 08.10.1999 US 158324 P
(43) Date of publication of application: 03.07.2002
(73) Proprietor: Pilling Weck Incorporated, Fort Washington, PA 19034 (US)
(72) Inventor: HOSSAIN, Mosaddeq, Somerville, NJ 08876 (US); BANIK, Robert, Long Valley, NJ 07853 (US)
(74) Representative: Gill, Ian Stephen
(86) International application number: PCT/US2000/027780
(87) International publication number: WO 2001/026565

(56) References cited:
- EP-A- 0 598 607
- US-A- 4 120 302
- US-A- 5 258 005
- US-A- 5 728 121

## Description

### Technical Field

This invention relates to surgery and more particularly to improvements in surgical grasping devices, for example forceps and clamps.

### Background Art

It has long been an objective of designers of surgical forceps, clamps and similar instruments to produce an instrument that can grasp slippery tissue securely and yet not cause trauma by puncturing, shearing, stretching or exerting excessive pressure on the tissue. To this end, numerous instruments have been designed having atraumatic toothed jaws made of metal. For an early example, see United States Patent 2,668,538, granted on February 9, 1954.

More recently, there has been a trend toward the use of non-metal gripping materials such, as polyvinyldiene fluoride, which are relatively smooth and pliable, but which exhibit sufficiently high friction that they are able to grasp wet, slippery tissue effectively. Such materials are particularly suited to use in disposable attachments to the jaws of grasping instruments, as described in United States Patent 5,728,121, dated March 17, 1998.

A trend toward minimally invasive surgery using laparoscopic instruments has given rise to efforts on the part of instrument designers to produce very small instrument jaws and disposable jaw attachments. The jaws and jaw attachments are desirably made as small as possible in size, so that they can pass through small incisions, or through very narrow passages in cannulae used in gaining access to various internal organs, vessels and other tissues.

Heretofore, practical jaw attachments have generally consisted of pliable, tissue-engaging pads mounted on bases of more rigid plastics. The bases have been designed to be removably engaged with instrument jaws, for example by snap-action latching devices which come into locking engagement with the instrument jaws when the attachments are slid into position. It is important that the attachments be very reliable, so that they do not become detached unintentionally and fall into the patient, where their retrieval may be difficult. Designers have encountered a minimum size limit, below which the plastics bases are insufficiently strong to be reliable. Below this minimum size limit, the latch mechanisms of the plastics bases may be inadequate, or the plastics bases may break into pieces. In either case, serious untoward consequences may result.

Alternative attachment base materials, metal, for example are not entirely satisfactory for very small jaw attachments for several reasons. First, when attached to a jaw, they can be quite difficult to remove. Second, unless the metal attachment bases are made to extremely close manufacturing tolerances, they may exhibit some "play" relative to the jaws, which is undesirable, especially in laparoscopic surgery, and are subject to accidental detachment from the jaws.

### Disclosure of Invention

This invention addresses the above-mentioned problems. Its principal object is to provide a jaw attachment which can be extremely small in size, and yet highly resistant to play, to breakage, and to inadvertent detachment from the instrument jaw on which it is mounted. Other objects of the invention include simplicity, reliability, ease of attachment and detachment, and simplicity of manufacture.

In accordance with the invention, a grasping attachment is removably mounted on at least a first jaw of an instrument having a pair of jaws capable of moving toward and away from each other. The first jaw has a surface facing toward the other jaw, a hole opening to the surface and a clip-engageable portion spaced from the hole. The grasping attachment comprises an assembly of a metal element and a plastics element, and also an elastomeric layer.

The metal element is in the form of a resilient sheet having a plate portion situated in facing relationship to the first jaw, a clip, unitary with the plate portion, and engaged with the clip-engageable portion of the first jaw, and an aperture in the plate portion. The aperture in the plate portion is aligned with the hole in the first jaw.

The plastics element comprises a substantially rigid plastics layer fixed to the plate portion of the metal element, with at least a part of the plate portion located between the plastics layer and the first jaw. The plastics element also has a projection extending from the plastics layer through the aperture in the plate portion. This projection either closely fits into the hole in the first jaw or fits into the hole with a snap fit.

The elastomeric layer is substantially less rigid than the plastics layer, and is secured to the assembly of the metal and plastics elements. At least a portion of the plastics layer is located between the elastomeric layer and the plate portion of the metal element.

The metal element lends strength and stiffness to the grasping attachment, allowing the dimensions of the attachment in the direction perpendicular to the surface of the first jaw to be minimized, and the projection of the plastics layer is sufficiently flexible to fit closely into the aperture of the first jaw without requiring excessively close manufacturing tolerances, or to fit into the aperture with a snap fit.

In a preferred version of the grasping device, the plate portion lies substantially in a plane and is elongated, having first and second ends and opposite longitudinal edges extending from the first end to the second end substantially in the direction of elongation. The clip comprises a pair of ears adjacent to the first end of the plate portion. These ears have first parts extending respectively from the opposite longitudinal edges of the plate portion, out of the plane of the plate portion. The ears also have second parts extending toward each other from the first parts. The second parts overlie the plate portion in spaced relation thereto, and the first jaw is received between the second parts and the plate portion.

The projection, which extends from the plastics layer, through the aperture in the plate portion and into the hole in the first jaw, is located closer to the second end of the plate portion than to the ears of the clip. In one embodiment, at least a portion of the projection extending beyond the plate portion has crushable ribs formed in its exterior wall. The crushable ribs are elongated in directions transverse to the plane of the plate portion, and are deformed by compression when the projection enters the hole in the jaw. The crushable ribs facilitate entry of the projection into the hole in the jaw, and establish a tight fit when the projection is situated in the hole. In another embodiment, the projection fits into the hole with a snap fit, in which case the ribs may be eliminated.

The substantially rigid plastics layer can extend laterally beyond the longitudinal edges and ends of the plate portion of the metal element. However, in a preferred embodiment, the combination of the plate portion of the metal element and the aperture therein overlies substantially the entire substantially rigid plastics layer. The longitudinal edges of the plate portion extend past the projection on opposite sides of the projection, and the plate portion is preferably shaped so that the distance between the longitudinal edges on opposite sides of the projection is greater than the distance between the longitudinal edges adjacent the location of the clip.

By virtue of the foregoing features, and especially the use of a metal element in combination with a plastics projection, the grasping attachment can be made sufficiently small to be used with jaw instruments designed to extend through cannulae having very small internal diameters, e.g. 5 mm or smaller.

Other objects, details and advantages of the invention will be apparent from the following detailed description when read in conjunction with the drawings.

### Brief Description of Drawings

FIG. 1 is a perspective view of a typical surgical grasping device, having jaws designed for use with grasping attachments;
FIG. 2 is a top plan view of the grasping attachment;
FIG. 3 is a longitudinal section of a grasping attachment taken on surface 3-3 of FIG. 2;
FIG. 4 is a sectional view of the grasping attachment taken on plane 4-4 in FIG. 3;
FIG. 5 is a fragmentary perspective view of the working end of a surgical grasping device of the kind shown in FIG. 1, with grasping attachments in accordance with the invention removably connected to both of its jaws; and
FIG. 6 is a sectional view, similar to FIG. 4, showing an alternative grasping attachment.

### Modes for Carryong Out the Invention

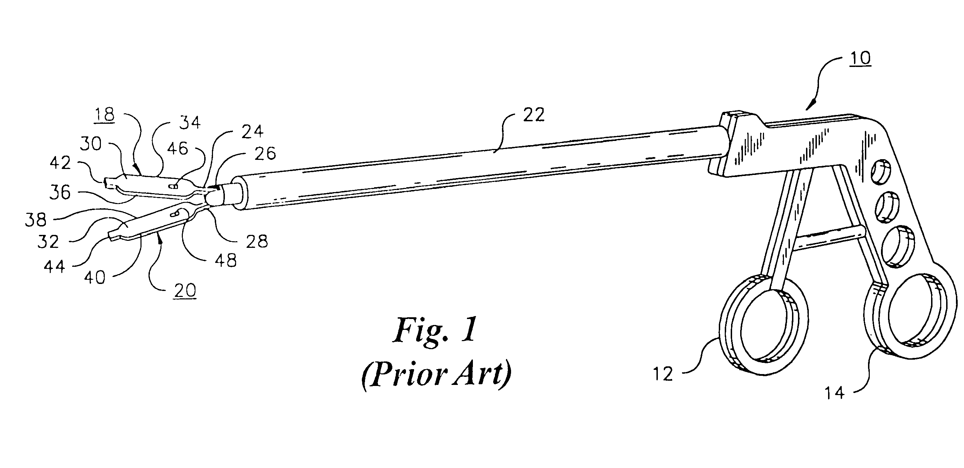

The instrument of FIG. 1 is a typical endoscopic forceps having a handle assembly 10 having a pair of ring handles 12 and 14, handle 12 being movable toward handle 14 to actuate the forceps jaws 18 and 20, which are situated at the opposite end of a barrel 22 from the handle. The direction from the handle assembly 10 toward the jaws will be referred to as the "distal" direction and the direction from the jaws toward the handle assembly will be referred to as the "proximal" direction.

In the preferred endoscopic forceps, a conventional linkage (not shown) located inside the handle and inside the barrel causes the jaws 18 and 20 to approach each other as the handle 12 is moved toward handle 14, and to move away from each other as the handle 12 is moved away from handle 14.

Although in most cases, both of the jaws move symmetrically relative to the barrel axis, it is possible to utilize alternative arrangements, for example, one in which only one of the jaws moves while the other remains in fixed relationship to the barrel.

As shown in FIG. 1, each of the jaws is generally in the form of an elongated metal plate extending distally from a stem. Thus, jaw 18 comprises a plate having a stem 24 extending through a slot 26 at the distal end of the barrel 22, and jaw 20 similarly comprises a plate having a stem 28 extending through slot 26. Each plate has inner and outer faces, the upper face 30 on jaw 18 being an outer face, and the upper face 32 on jaw 20 being an inner face. Each of the plates has a central portion having parallel edges, the parallel edges of the plate of jaw 18 being seen at 34 and 36, and the parallel edges_of the plate of jaw 20 being seen at 38 and 40. The inner faces of the jaws are in opposed relationship to each other, and can be brought into a relationship with each other such that the inner faces are parallel to each other and spaced from each other by a distance of about 2 mm.

The plate of each jaw is tapered adjacent to its distal end and has a tip comprising parallel edges which are closer to each other than the parallel edges of the main portion of the plate. These tips are adapted to be engaged by a C-shaped clip of an attachment which will be described below. Thus jaw 18 has a tip 42, and jaw 20 has a tip 44.

The inner face of the plate of each jaw is also provided with a hole near its proximal end for receiving a projection of an attachment, as will be described. The holes are preferably, but not necessarily, through holes, hole 46 being a through hole in jaw 18 and hole 48 being a through hole in jaw 20.

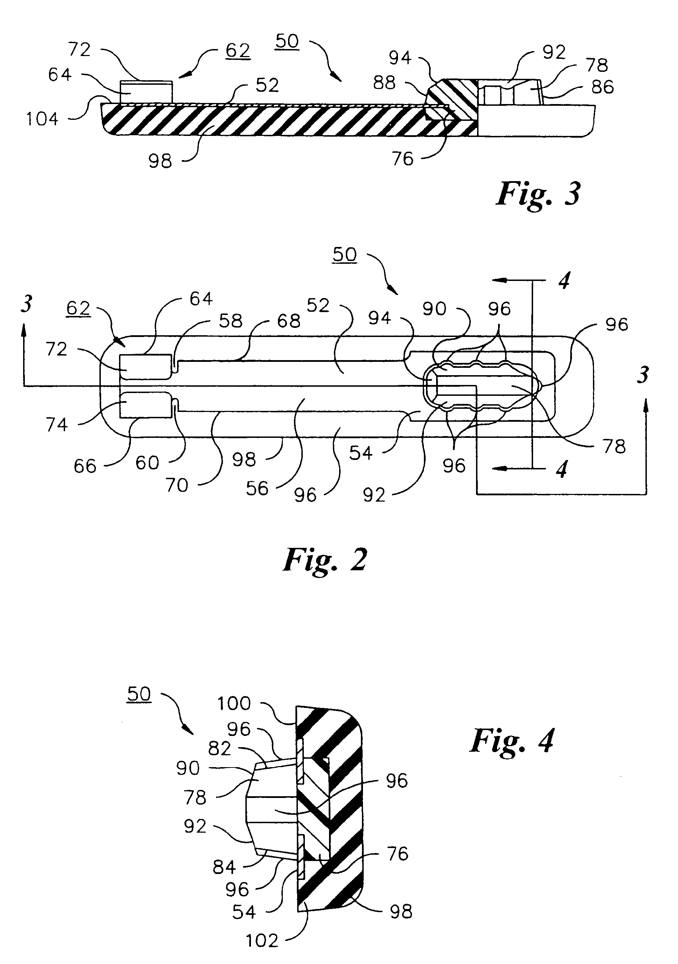

The grasping attachments, which will be described with reference to FIGs. 2-5, are intended to provide a secure, but atraumatic, grip on anatomical tissue. The tissue-grasping components of the attachments are situated on the inner faces of the jaws 18 and 20. C-shaped clips, which constitute components of the attachments fit closely onto the tips 42 and 44, while projections on the attachments fit into holes 46 and 48. The attachments have resilience allowing them to be bent away from the planes of the inner faces of the jaws when the C-shaped clips are attached to the tips 42 and 44. It is this same resilience which urges the projections into the holes when the C-shaped clips are engaged with the tips, thereby securing the attachments to the jaws and preventing unintended detachment, while allowing the attachments to be removed readily by deliberate manipulation.

As shown in FIGs. 2, 3 and 4, the grasping attachment 50 is elongated and generally rectangular in shape. It comprises an elongated sheet metal element having a plate portion 52 with a wide part 54 adjacent to a proximal end of the attachment, a narrower part 56 extending from the wide part toward the distal end, and notches 58 and 60 spaced a short distance from the distal end. On the distal side of the notches adjacent to the distal end of the grasping attachment, the sheet metal element has ears which form a C-shaped clip 62. The ears comprise first parts 64 and 66, which extend respectively, from opposite longitudinal edges 68 and 70 of the plate, out of the plane in which plate portion 52 lies. At the upper ends of parts 64 and 66, the ears have second parts 72 and 74, which extend toward each other from the first parts and overlie the plate portion 52 in spaced relation thereto. The size of the C-shaped clip 62 is such that it can be closely fitted onto one or the other of projections 42 and 44 in FIG. 1. The notches 58 and 60 facilitate bending of the plate portion of the metal element at a location adjacent to the C-shaped clip so that the grasping element can be readily installed on, and removed from, and instrument jaw.

The sheet metal element is preferably composed of a stainless steel having enough resilience to enable the C-shaped clip to grip projection 42 or 44 tightly, and to enable the narrow part 56 of the sheet metal element to bend for connection and removal of the attachment.

A layer 76 (FIGs. 3 and 4) is situated adjacent the wide part 54 of plate portion 52 of the sheet metal element. This layer is a substantially rigid layer, composed of a plastics material, preferably polypropylene, or another similar synthetic resin suitable for use in a surgical instrument. The layer 76 is a part of a molded element including a projection 78 which extends outwardly from the wide part 54 plate portion 52. The element is molded so that the margins of layer 76 underlie the plate portion and the projection overlies the plate portion, and consequently, the plastics element is permanently attached to the metal plate portion.

The projection is slightly tapered, having sloping sidewalls 82 and 84, as well as sloping front and rear ends 86 and 88, respectively. The top of the projection is beveled at 90, 92 and 94 to facilitate entry of the projection into hole 46 or 48, and the proximal and side walls are provided with deformable ribs 96, which, as mentioned previously, both facilitate entry of the projection into the hole in the instrument jaw and ensure a tight fit so that the grasping attachment is held firmly on the jaw.

An elastomeric pad 98, preferably made of silicone rubber, is secured to the plastics layer 76, and to the plate portion of the metal element, preferably by an adhesive (not shown). The face of the plate portion which is contacted by the pad may be etched for more effective adhesion. The pad is shaped with a rim so that it forms a recess receiving the plate portion 52. Side portions of the rim are seen in FIG. 4 at 100 and 102, and a distal portion of the rim is seen in FIG. 3 at 104.

The exposed surface of the elastomeric pad is covered by a membrane which imparts to it the ability to hold anatomical tissue effectively without causing trauma. A variety of suitable materials can be used, for example, a high-density non-woven polyethylene available under the trademark TYVEK, polymers such as polyvinyldiene fluoride (PVDF), and spunbonded materials such as a polyester membrane material, e.g. Ahlstorm's 3283 or Reemay's 2040 product. Other suitable materials are described in U.S. Patent 5,728,121.

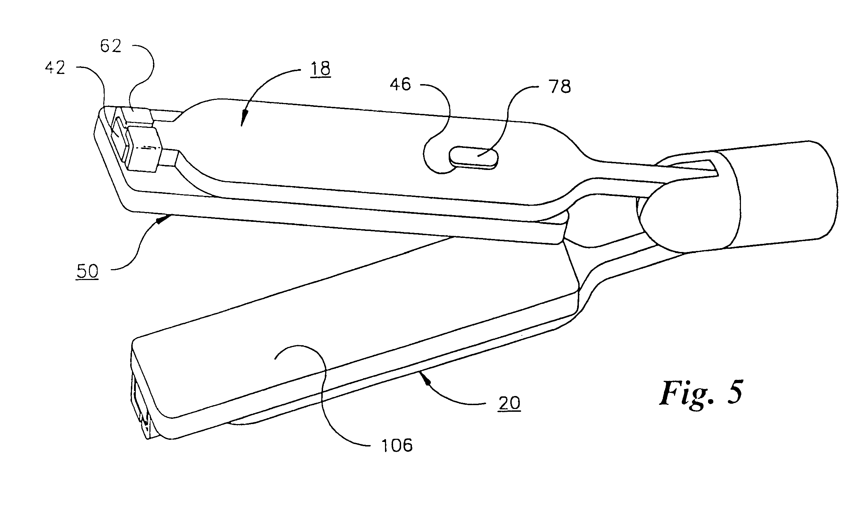

As shown in FIG. 5, grasping attachment 50 is attached to a jaw 18 by engaging its C-shaped clip 62 with projection 42 of the jaw, and allowing projection 78 to enter hole 46 of the jaw. The spring action of the metal element holds the projection in hole 46 so that the grasping attachment is firmly secured in place on the jaw. An identical grasping attachment 106 may be secured to jaw 20 in a similar manner, so that the elastomeric pads of the two grasping attachments are in opposed relationship to each other.

The grasping attachments may be supplied pre-sterilized in sealed packages, and readily attached to the instrument of FIG. 1 prior to surgery. After use, the attachments can be removed from the jaws by bending the attachments until the projections clear the holes 46 and 48 and sliding the attachments in the distal direction until the C-shaped clips disengage the end projections 42 and 44 on the jaws. The attachments can then be discarded.

The invention makes it possible to provide extremely small, but strong and reliable grasping attachments suitable for laparoscopic surgery using cannulae having internal diameters of 5 mm or smaller.

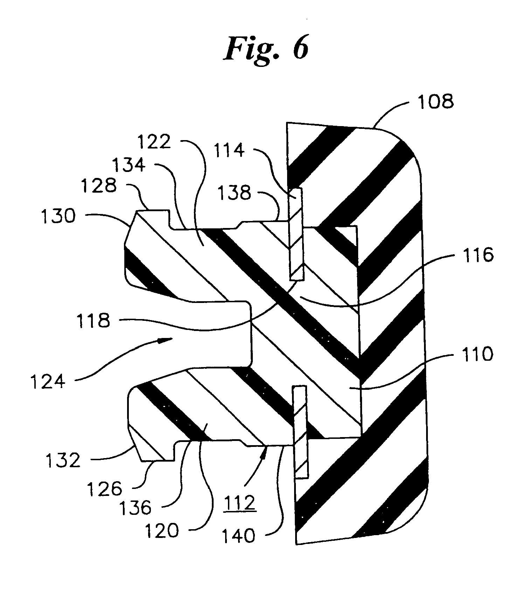

As mentioned previously, as an alternative, the projection can be made to fit into hole 46 or 48 by a snap fit. This alternative will be described with reference to FIG. 6, in which an elastomeric pad 108, of silicone rubber or similar material, is secured to a layer 110 of a resilient plastics element 112 and also to metal plate 114. The plastics element 112 has a narrow portion 116, which extends through a hole 118 in the metal plate, and is generally similar to the structure depicted in FIGs. 3 and 4 except that the portion of the plastics element which engages the hole 46 or 48 in the instrument jaws 18 or 20 comprises two elements 120 and 122, having a gap 124 between them and outwardly projecting flanges 126 and 128, which engage the outer faces of the jaws to hold the grasping element firmly in place.

As seen in FIG. 6, the elements 120 and 122 are beveled at 130 and 132 so that the force exerted in pressing the plastics element 112 into a hole 46 or 48 pushes elements 120 and 122 toward each other, allowing the flanges 126 and 128 to pass through the hole. The outer faces of element 112 are configured so that the element 112 is narrower at locations 134 and 136 adjacent the flanges than at locations 138 and 140 adjacent the metal plate 114. The narrower portion of the element 112 ensure that its flanges can reliably engage the outer face of the jaw with to which it is attached with a snap fit. The wider portion at the base of element 112 fits snugly into hole 46 or 48 in the jaw to prevent movement of the grasping pad relative to the jaw. The embodiment of FIG. 6 does not rely upon resilience of the metal plate 114 for secure attachment of the grasping pad to the instrument jaws, and is a preferred embodiment for most applications.

Various other modifications can be made to the grasping attachment described. For example, the plastics layer can be made larger, and can even be made coextensive with the elastomeric layer, in which case the elastomeric layer is attached primarily to the plastics layer. Additional molded projections, which fit into holes in the metal layer but do not extend beyond the metal layer, can be used to supplement a projection corresponding to projection 78 in securing the plastics layer to the metal layer.

Still other modifications may be made to the apparatus and method described above without departing from the scope of the invention as defined in the following claims.

## Claims

1. A surgical grasping device comprising:
a grasping attachment removably mountable on the jaw of a surgical instrument, the grasping attachment comprising an assembly of a metal element and a plastics element;
the metal element being in the form of a resilient sheet having a plate portion (52, 114), a clip (62), unitary with the plate portion (52, 114), and engageable with a clip-engageable portion of an instrument jaw, and an aperture, in the plate portion (52, 114), and alignable with a hole in an instrument jaw; and
the plastics element comprising a substantially rigid plastics layer (76, 110) fixed to the plate portion (52, 114) of the metal element, with at least a part of the plate portion (52, 114) being locatable between the plastics layer (76, 110) and an instrument jaw, the plastics element having a projection (78, 120, 122) extending from said plastics layer (76, 110) through the aperture in the plate portion (52, 114), and beyond the plate portion (52 , 114), for entry into a hole in the instrument jaw; and
an elastomeric layer (98, 108), substantially less rigid than said plastics, layer (76, 110), secured to said assembly of said metal element and said plastics element, at least a portion of the plastics layer (76, 110) being located between the elastomeric layer (98, 108) and the plate portion (52, 114) of the metal element;
whereby the metal element lends strength and stiffness to the grasping attachment, allowing the dimensions of the attachment in directions perpendicular to said sheet portion to be minimized; and the projection (78, 120, 122) of the plastics layer (76, 110) is sufficiently flexible to fit closely into an aperture of a jaw without requiring excessively close manufacturing tolerances.

2. A surgical grasping device according to claim 1, in which the plate portion (52, 114) lies substantially in a plane and is elongated, having first and second ends and opposite longitudinal edges extending from the first end to the second end substantially in the direction of elongation, and in which the clip (62) comprises a pair of ears adjacent to said first end, the ears having first parts (64, 66) extending respectively from the opposite longitudinal edges out of the plane of the plate portion (52), and second parts (72, 74) extending toward each other from the first parts (64, 66), the second parts (72, 74) overlying the plate portion (52, 114) in spaced relation thereto.

3. A surgical grasping device comprising:
first and second jaws (18, 20) mounted for movement toward and away from each other, the first jaw (20) being a rigid jaw having a surface (32) facing toward the second jaw, a hole (48) opening to said surface and
a clip-engageable portion (44) spaced from said hole; a grasping attachment removably mounted on said first jaw, the grasping attachment comprising an assembly of a metal element and a plastics element;
the metal element being in the form of a resilient sheet having a plate portion (52, 114) situated in facing relationship to the first jaw, a clip (62), unitary with the plate portion (52, 114), and engaged with the clip-engageable portion of the first jaw, and an aperture, in the plate portion (52, 114), and aligned with said hole in the first jaw; and
the plastics element comprising a substantially rigid plastics layer (76, 110) fixed to the plate portion (52, 114) of the metal element, with at least a part of the plate portion (52, 114) being located between the plastics layer (76, 110) and the first jaw, the plastics element having a projection (78, 120, 122) extending from said plastics layer (76, 110) through the aperture in the plate portion (52, 114) and closely fitting into said hole (48) in the first jaw; and
an elastomeric layer (98, 108), substantially less rigid than said plastics layer (76, 110), secured to said assembly of a said metal element and said plastics element, at least a portion of the plastics layer (76, 110) being located between the elastomeric layer (98, 108) and the plate portion (52, 114) of the metal element;
whereby the metal element lends strength and stiffness to the grasping attachment, allowing the dimensions of the attachment in the direction perpendicular to said surface of the first jaw to be minimized; and the projection (78, 120, 122) of the plastics layer (76, 110) is sufficiently flexible to fit closely into the aperture of the first jaw without requiring excessively close manufacturing tolerances.

4. A surgical grasping device according to claim 3, in which the plate portion (52, 114) lies substantially in a plane and is elongated, having first and second ends and opposite longitudinal edges extending from the first end to the second end substantially in the direction of elongation, and in which the clip (62) comprises a pair of ears adjacent to said first end, the ears having first parts (64, 66) extending respectively from the opposite longitudinal edges out of the plane of the plate portion (52, 114), and second parts (72, 74) extending toward each other from the first parts (64, 66), the second parts (72, 74) overlying the plate portion (52, 114) in spaced relation thereto, and the first jaw being received between said second parts (72, 74) and said plate portion (52, 114).

5. A surgical grasping device according to claim 2 or 4, in which the projection (78, 120, 122) is located closer to said second end of the plate portion (52) than to said ears.

6. A surgical grasping device according to any one of the preceding claims, in which the plate portion (52) lies substantially in a plane, and in which at least a portion of the projection (78) of the plastics element extending beyond the plate portion (52) has an exterior wall with crushable ribs (96) formed therein, the crushable ribs (96) being elongated in directions transverse to the plane of said plate portion (52).

7. A surgical grasping device according to any one of claims 1 to 5, in which the plate portion (114) lies substantially in a plane, and in which at least a portion of the projection (120, 122) of the plastics element extending beyond the plate portion (114) has an exterior wall with laterally projecting flanges (126, 128), spaced from said plate portion (114), for securing the grasping attachment to a jaw with a snap fit.

## Patentansprüche

1. Chirurgische Greifvorrichtung, enthaltend:
einen Greifaufsatz, der abnehmbar an der Klemmbacke eines chirurgischen Instrumentes angebracht werden kann, wobei der Greifaufsatz eine Anordnung eines Metallelementes und eines Kunststoffelementes enthält;
wobei das Metallelement die Gestalt eines federnden Bleches hat, das einen Plattenabschnitt (52, 114), einen Bügel (62), der integraler Bestandteil des Plattenabschnittes (52, 114) ist und mit einem mit dem Bügel in Eingriff bringbaren Abschnitt einer Instrumentenklemmbacke in Eingriff gebracht werden kann, und eine Öffnung im Plattenabschnitt (52, 114) aufweist, die mit einem Loch in einer Instrumentenklemmbacke ausgerichtet werden kann; und
das Kunststoffelement eine im wesentlichen starre Kunststoffschicht (76, 110) enthält, die am Plattenabschnitt (52, 114) des Metallelementes fixiert ist, wobei wenigstens ein Teil des Plattenabschnittes (52, 114) zwischen der Kunststoffschicht (76, 110) und einer Instrumentenklemmbacke angeordnet werden kann, wobei das Kunststoffelement einen Vorsprung (78, 120, 122) hat, der sich von der Kunststoffschicht (76, 110) durch die Öffnung im Plattenabschnitt (52, 144) und über den Plattenabschnitt (52, 114) hinaus erstreckt, um in ein Loch in der Instrumentenklemmbacke einzutreten; und
eine Elastomerschicht (98, 108), die im wesentlichen weniger starr ist als die Kunststoffschicht (76, 110) und an der Anordnung des Metallelementes und des Kunststoffelementes angebracht ist, wobei sich wenigstens ein Abschnitt der Kunststoffschicht (76, 110) zwischen der Elastomerschicht (98, 108) und dem Plattenabschnitt (52, 114) des Metallelementes befindet;
wobei das Metallelement dem Greifaufsatz Festigkeit und Steifigkeit verleiht, wodurch die Abmessungen des Aufsatzes in den Richtungen senkrecht zum Blechabschnitt minimiert werden können, und der Vorsprung (78, 120, 122) der Kunststoffschicht (76, 110) ausreichend flexibel ist, um eng in eine Öffnung einer Klemmbacke zu passen, ohne dass übermäßig geringe Fertigungstoleranzen erforderlich sind.

2. Chirurgische Greifvorrichtung nach Anspruch 1, bei der der Plattenabschnitt (52, 114) im wesentlichen in einer Ebene liegt und länglich ist und über ein erstes und ein zweites Ende sowie gegenüberliegende Längskanten verfügt, die vom ersten Ende zum zweiten Ende im wesentlichen in der Längsrichtung verlaufen, und bei der der Bügel (62) zwei Laschen benachbart dem ersten Ende enthält, wobei die Laschen erste Teile (64, 66), die sich jeweils von den gegenüberliegenden Längskanten aus der Ebene des Plattenabschnittes (52) erstrecken, und zweite Teile (72, 74) enthalten, die sich von den ersten Teilen (64, 66) aufeinander zu erstrecken, wobei die zweiten Teile (72, 74) über dem Plattenabschnitt (52, 114) in einem Abstand zu diesem liegen.

3. Chirurgische Greifvorrichtung enthaltend:
eine erste und eine zweite Klemmbacke (18, 20), die für eine Bewegung aufeinander zu und voneinander weg angebracht sind, wobei die erste Klemmbacke (20) eine starre Klemmbacke ist, die eine Oberfläche (32), die der zweiten Klemmbacke zugewandt ist, ein Loch (48), das sich zur Oberfläche öffnet, und einen mit dem Bügel in Eingriff bringbaren Abschnitt (44) aufweist, der zu diesem Loch mit Abstand angeordnet ist;
einen Greifaufsatz, der abnehmbar an der ersten Klemmbacke angebracht ist, wobei der Greifaufsatz eine Anordnung aus einem Metallelement und einem Kunststoffelement enthält;
wobei das Metallelement die Gestalt eines federnden Bleches hat, das einen Plattenabschnitt (52, 114), der gegenüberliegend zur ersten Klemmbacke angeordnet ist, einen Bügel (62), der integraler Bestandteil des Plattenabschnittes (52, 114) ist und mit dem mit dem Bügel in Eingriff bringbaren Abschnitt der ersten Klemmbacke in Eingriff steht, sowie eine Öffnung im Plattenabschnitt (52, 114) aufweist, die mit dem Loch in der ersten Klemmbacke ausgerichtet ist; und
das Kunststoffelement eine im wesentlichen starre Kunststoffschicht (76, 110) enthält, die am Plattenabschnitt (52, 114) des Metallelementes fixiert ist, wobei sich wenigstens ein Teil des Plattenabschnittes (52, 114) zwischen der Kunststoffschicht (76, 110) und der ersten Klemmbacke befindet, wobei das Kunststoffelement einen Vorsprung (78, 120, 122) aufweist, der sich von der Kunststoffschicht (76, 110) durch die Öffnung im Plattenabschnitt (52, 114) erstreckt und eng in das Loch (48) in der ersten Klemmbacke passt; und
eine Elastomerschicht (98, 108), die im wesentlichen weniger starr ist als die Kunststoffschicht (76, 110) und an der Anordnung des Metallelementes und des Kunststoffelementes befestigt ist, wobei sich wenigstens ein Abschnitt der Kunststoffschicht (76, 110) zwischen der Elastomerschicht (98, 108) und dem Plattenabschnitt (52, 114) des Metallelementes befindet;
wobei das Metallelement dem Greifaufsatz Festigkeit und Steifigkeit verleiht, wodurch die Abmessungen des Aufsatzes in der Richtung senkrecht zur Oberfläche der ersten Klemmbacke minimiert werden können und der Vorsprung (78, 120, 122) der Kunststoffschicht (76, 110) ausreichend flexibel ist, um eng in die Öffnung der ersten Klemmbacke zu passen, ohne dass übermäßig geringe Fertigungstoleranzen erforderlich sind.

4. Chirurgische Greifvorrichtung nach Anspruch 3, bei der der Plattenabschnitt (52, 114) im wesentlichen in einer Ebene liegt und länglich ist und über ein erstes und ein zweites Ende sowie gegenüberliegende Längskanten verfügt, die sich vom ersten Ende zum zweiten Ende im wesentlich in Längsrichtung erstrecken, und bei der der Bügel (62) zwei Laschen benachbart zum ersten Ende enthält, wobei die Laschen erste Teile (64, 66), die sich jeweils von den gegenüberliegenden Längskanten aus der Ebene des Plattenabschnittes (52, 114) erstrecken, und zweite Teile (72, 74) aufweisen, die sich von den ersten Teilen (64, 66) aufeinander zu erstrecken, wobei die zweiten Teile (72, 74) über dem Plattenabschnitt (52, 114) in einem Abstand zu diesem liegen und die erste Klemmbacke zwischen den zweiten Teilen (72, 74) und dem Plattenabschnitt (52, 114) aufgenommen ist.

5. Chirurgische Greifvorrichtung nach Anspruch 2 oder 4, bei der der Vorsprung (78, 120, 122) dichter am zweiten Ende des Plattenabschnittes (52) als an den Laschen angeordnet ist.

6. Chirurgische Greifvorrichtung nach einem der vorhergehenden Ansprüche, bei der der Plattenabschnitt (52) im wesentlichen in einer Ebene liegt, und bei der wenigstens ein Abschnitt des Vorsprungs (78) des Kunststoffelementes, der sich über den Plattenabschnitt (52) hinaus erstreckt, eine Außenwand mit darin ausgebildeten eindrückbaren Rippen (96) aufweist, wobei die eindrückbaren Rippen (96) in Richtungen quer zur Ebene des Plattenabschnittes (52) ihre Längsausdehnung haben.

7. Chirurgische Greifvorrichtung nach einem der Ansprüche 1 bis 5, bei der der Plattenabschnitt (114) im wesentlichen in einer Ebene liegt, und bei der wenigstens ein Abschnitt des Vorsprungs (120, 122) des Kunststoffelementes,'der sich . über den Plattenabschnitt (114) hinaus erstreckt, eine Außenwand mit seitlich hervorragenden Flanschen (126, 128) aufweist, die vom Plattenabschnitt (114) mit Abstand angeordnet sind und den Greifaufsatz an einer Klemmbacke durch Einrasten- befestigen.

## Revendications

1. Dispositif chirurgical de préhension comprenant:
un accessoire de préhension pouvant être monté de façon amovible sur la mâchoire d'un instrument chirurgical, l'accessoire de préhension comprenant un assemblage d'un élément métallique et d'un élément en plastique,
l'élément métallique étant sous la forme d'une feuille élastique ayant une partie de plaque (52, 114), un clip (62), unitaire avec la partie de plaque (52, 114) et emboîtable dans une partie, emboîtable par un clip, d'une mâchoire d'un instrument, et une ouverture, dans la partie de plaque (52, 114), et alignable avec un orifice dans une mâchoire d'un instrument ; et
l'élément en plastique comprenant une couche de plastique (76, 110) essentiellement rigide fixée à la partie de plaque (52, 114) de l'élément métallique, avec au moins une portion de la partie de plaque (52, 114) pouvant être située entre la couche de plastique (76, 110) et une mâchoire d'un instrument, l'élément en plastique ayant une saillie (78, 120, 122) s'étendant à partir de ladite couche de plastique (76, 110) à travers une ouverture dans la partie de plaque (52, 114), et au-delà de la partie de plaque (52, 114), pour entrer dans un orifice dans la mâchoire de l'instrument ; et
une couche élastomère (98, 108), essentiellement moins rigide que ladite couche de plastique (76, 110), fixée audit ensemble dudit élément métallique et dudit élément en plastique, au moins une partie de la couche de plastique (76, 110) étant située entre la couche élastomère (98, 108) et la partie de plaque (52, 114) de l'élément métallique ;
où l'élément métallique confère une résistance et une rigidité à l'accessoire de préhension, permettant que les dimensions de l'accessoire dans les directions perpendiculaires à ladite partie de feuille soient minimisées ; et la saillie (78, 120, 122) de la couche de plastique (76, 110) est suffisamment flexible pour s'emboîter étroitement dans une ouverture d'une mâchoire sans nécessiter des tolérances de fabrication excessivement étroites.

2. Dispositif chirurgical de préhension selon la revendication 1, dans lequel la partie de plaque (52, 114) se trouve essentiellement dans un plan et est allongée, ayant une première et une seconde extrémités et des bords longitudinaux opposés s'étendant à partir de la première extrémité jusqu'à la seconde extrémité essentiellement dans la direction de l'allongement, et dans lequel le clip (62) comprend une paire de pattes adjacentes à ladite première extrémité, les pattes ayant des premières parties (64, 66) s'étendant respectivement à partir des bords longitudinaux opposés hors du plan de la partie de plaque (52), et des secondes parties (72, 74) s'étendant l'une vers l'autre à partir des premières parties (64, 66), les secondes parties (72, 74) étant superposées à la partie de plaque (52, 114) dans une relation espacée avec celle-ci.

3. Dispositif chirurgical de préhension comprenant :
une première et une seconde mâchoires (18, 20) montées de façon à permettre un mouvement de l'une vers l'autre, la première mâchoire (20) étant une mâchoire rigide ayant une surface (32) faisant face à la seconde mâchoire, un orifice (48) s'ouvrant sur ladite surface et une partie (44) emboîtable par un clip espacée dudit orifice ;
un accessoire de préhension monté de façon amovible sur ladite première mâchoire, l'accessoire de préhension comprenant un ensemble d'un élément métallique et d'un élément en plastique ;
l'élément métallique étant sous la forme d'une feuille élastique ayant une partie de plaque (52, 114) située dans une relation de face par rapport à la première mâchoire, un clip (62) unitaire avec la partie de plaque (52, 114) et emboîté dans la partie emboîtable par un clip de la première mâchoire, et une ouverture, dans la partie de plaque (52, 114), et alignée avec ledit orifice dans la première mâchoire ; et
l'élément en plastique comprenant une couche de plastique (76, 110) essentiellement rigide fixée à la partie de plaque (52, 114) de l'élément métallique, avec au moins une partie de la partie de plaque (52, 114) étant située entre la couche de plastique (76, 110) et la première mâchoire, l'élément en plastique ayant une saillie (78, 120, 122) s'étendant à partir de ladite couche de plastique (76, 110) à travers l'ouverture dans la partie de plaque (52, 114) et s'emboîtant étroitement dans ledit orifice (48) dans la première mâchoire ; et
une couche élastomère (98, 108), essentiellement moins rigide que ladite couche de plastique (76, 110), fixée audit ensemble dudit élément métallique et dudit élément en plastique, au moins une partie de la couche de plastique (76, 110) étant située entre la couche élastomère (98, 108) et la partie de plaque (52, 114) de l'élément métallique ;
moyennant quoi l'élément métallique confère une résistance et une rigidité à l'accessoire de préhension, permettant que les dimensions de l'accessoire dans la direction perpendiculaire à ladite surface de la première mâchoire soient minimisées ; et la saillie (78, 120, 122) de la couche de plastique (76, 110) est suffisamment flexible pour s'emboîter étroitement dans l'ouverture de la première mâchoire sans nécessiter des tolérances de fabrication excessivement étroites.

4. Dispositif chirurgical de préhension selon la revendication 3, dans lequel la partie de plaque (52, 114) se trouve essentiellement dans un plan et est allongée, ayant une première et une seconde extrémités et des bords longitudinaux opposés s'étendant à partir de la première extrémité jusqu'à la seconde extrémité essentiellement dans la direction de l'allongement, et dans lequel le clip (62) comprend une paire de pattes adjacentes à ladite première extrémité, les pattes ayant des premières parties (64, 66) s'étendant respectivement à partir des bords longitudinaux opposés hors du plan de la partie de plaque (52, 114), et des secondes parties (72, 74) s'étendant l'une vers l'autre à partir des premières parties (64, 66), les secondes parties (72, 74) étant superposées à la partie de plaque (52, 114) dans une relation espacée avec celle-ci, et la première mâchoire étant reçue entre lesdites secondes parties (72, 74) et ladite partie de plaque (52, 114).

5. Dispositif chirurgical de préhension selon la revendication 2 ou 4, dans lequel la saillie (78, 120, 122) est située plus près de ladite seconde extrémité de la partie de plaque (52) que desdites pattes.

6. Dispositif chirurgical de préhension selon l'une quelconque des revendications précédentes, dans lequel la partie de plaque (52) se trouve essentiellement dans un plan, et dans lequel au moins une partie de la saillie (78) de l'élément en plastique s'étendant au-delà de la partie de plaque (52) a une paroi extérieure avec des ailettes écrasables (96) formées dans celle-ci, les ailettes écrasables (96) étant allongées dans des directions transversales au plan de ladite partie de plaque (52).

7. Dispositif chirurgical de préhension selon l'une quelconque des revendications 1 à 5, dans lequel la partie de plaque (114) se trouve essentiellement dans un plan, et dans lequel au moins une partie de la saillie (120, 122) de l'élément en plastique s'étendant au-delà de la partie de plaque (114) a une paroi extérieure avec des épaulements (126, 128) faisant saillie latéralement, espacés de ladite partie de plaque (114), destinés à assembler l'accessoire de préhension à une mâchoire par un emboîtement-pression.
